(19) Europäisches Patentamt
European Patent Office
Office européen des brevets

(11) Numéro de publication: **0 126 685**
**A1**

(12) # DEMANDE DE BREVET EUROPEEN

(21) Numéro de dépôt: **84400984.5**

(22) Date de dépôt: **15.05.84**

(51) Int. Cl.³: **C 07 C 103/12**, C 07 H 15/24, A 61 K 37/02

(30) Priorité: **16.05.83 FR 8308051**

(43) Date de publication de la demande: **28.11.84**
**Bulletin 84/48**

(84) Etats contractants désignés: **AT BE CH DE GB IT LI LU NL SE**

(71) Demandeur: **Etablissement Public dit: CENTRE NATIONAL DE LA RECHERCHE SCIENTIFIQUE (CNRS), 15, Quai Anatole France, F-75007 Paris (FR)**

(72) Inventeur: **Monsigny, Michel, 341, rue des Bouvreuils, F-45590 Saint Cyr en Val (FR)**
Inventeur: **Mayer, Roger, 11, Allée des Chênes, F-45100 Orleans (FR)**

(74) Mandataire: **Nony, Michel, Cabinet Nony 29, rue Cambacérès, F-75008 Paris (FR)**

(54) **Nouveaux dérivés acylés hydrosolubles de peptides ou d'amino-acides, leur préparation et leur application.**

(57) Nouveaux dérivés acylés hydrosolubles de peptides, d'amino-acides, ou de dérivés de peptides ou d'amino-acides à groupement C-terminal modifié; leur préparation et leur application, notamment à l'obtention de dérivés hydrosolubles d'acides aminés ou de peptides à groupement C-terminal modifié ou non.

EP 0 126 685 A1

Nouveaux dérivés acylés hydrosolubles de peptides ou d'amino-acides, leur préparation et leur application.

La présente invention a pour objet de nouveaux dérivés acylés hydrosolubles de peptides ou d'amino-acides, leur préparation et leur application.

On sait que le dosage colorimétrique ou fluorimétrique des protéases (ou peptidases) utilise divers dérivés d'acides aminés ou de peptides dont l'extrémité N-terminale est substituée par un groupement acétyle, benzoyle, t-butyloxycarbonyle, benzyloxycarbonyle, glutaryle, succinyle, tosyle, etc..., et dont l'extrémité C-terminale est amidée par un groupement nitroanilide, 4-méthoxy-2-naphtylamine, amino-7-méthyl-4-coumarine, alpha-naphtylamine, beta-naphtylamine, 3-amino-9-éthyl-carbazole, indolylamine, 3-amino-5-bromoindole, etc..., ou estérifiée par un groupement alpha- ou beta-naphtol, naphtol AS-D (2-méthoxyanilide de l'acide 3-hydroxy-2-naphtoïque,) etc... Ces mêmes dérivés peuvent être utilisés pour visualiser les protéases et les peptidases *in situ* par les méthodes de l'histochimie enzymatique (Lojda, Z., Gossrau, R. et Schiebler, T.H. (1979) Springer-Verlag, Berlin, pp 339). Généralement, ces dérivés sont peu solubles dans l'eau et il est nécessaire de les dissoudre au préalable dans un solvant organique et ensuite de diluer ces solutions dans un tampon. La faible solubilité des substrats entraîne une faible sensibilité des méthodes de dosage des protéases et des peptidases et limite les possibilités de visualisation par des méthodes histochimiques.

On sait également que certains dérivés d'amino-acides ou de peptides peuvent jouer le rôle d'inhibiteur d'enzymes et être utilisés notamment comme médicaments, par exemple comme substrats-suicides pour lesdites enzymes. Ici encore, la préparation, la mise sous forme galénique appropriée et l'utilisation *in vitro* ou *in vivo* de ces dérivés sont rendues difficiles en raison de leur faible solubilité dans l'eau.

On sait enfin que l'on peut obtenir des prodrogues, permettant de conférer notamment un effet retard ou de faciliter le transport de drogue *in vivo* en liant la drogue (substance active) à un peptide, mais leur faible solubilité dans l'eau constitue souvent un handicap pour la préparation et l'efficacité de ces prodrogues.

De telles prodrogues sont intéressantes en particulier lorsque le peptide constitue un bon substrat pour une protéase spécifique sécrétée par la cible de la drogue active, (par exemple par une cellule tumorale ou par un micro-organisme pathogène), car dans ce cas la drogue active n'est libérée que dans l'environnement de la cible, ce qui a pour effet de favoriser l'efficacité de la drogue.

La présente invention a pour objet de nouveaux dérivés acylés

hydrosolubles de peptides, d'amino-acides, ou de peptides ou d'amino-acides à groupement C-terminal modifié, caractérisés par le fait que leur groupement N-terminal est acylé par un groupement polyhydroxyalcanoyle.

Ledit groupement polyhydroxyalcanoyle possède généralement 3 à 10 atomes de carbone et au moins 2 groupements hydroxyles.
L'invention s'étend notamment aux dits dérivés acylés de peptides ou d'amino-acides dont le groupement C-terminal porte un substituant -X tel que défini ci-après.

De préférence, ledit groupement polyhydroxyalcanoyle (également désigné par l'abréviation PHA) répond à la formule $R_1 - (CHOH)_m - CO -$, dans laquelle $R_1$ représente H - ou $CH_3$ - et m est un nombre entier pouvant varier de 2 à 7.

L'invention a en particulier pour objet les nouveaux dérivés de formule générale I:

$$R_1-(CHOH)_m-CO-(NH-\underset{\displaystyle R}{\underset{|}{C}}H-CO)_n-X \qquad (I)$$

dans laquelle $R_1$ et m sont définis comme précédemment, R est une chaîne latérale d'amino-acides, n est un nombre entier pouvant varier de 1 à 10 et X est un groupement actif tel que par exemple un substituant capable de conférer aux dérivés de formule I une coloration, une fluorescence ou une activité pharmacologique, étant entendu que lorsque n est supérieur à 1, les substituants R peuvent être différents dans un même composé de formule I.

Parmi les composés de l'invention on citera par exemple ceux pour lesquels $R_1-(CHOH)_m-CO-$ représente un reste dihydroxypropionyle, érythronoyle, thréonoyle, ribonoyle, arabinoyle, xylonoyle, lyxonoyle, gluconoyle, galactonoyle, mannonoyle, glycoheptonoyle, glycooctonoyle, etc...; R représente la chaîne latérale d'amino-acides D ou L, en particulier d'amino-acides de la série L, choisis parmi les amino-acides suivants: sérine, thréonine, asparagine, proline, glutamine, glycine, alanine, valine, cystéine ou un de ses dérivés S-substitués, méthionine, leucine, isoleucine, phénylalanine, p-nitrophénylalanine, tyrosine ou un de ses dérivés O-substitués, lysine, ornithine, arginine, tryptophane, acide aspartique, acide glutamique, gamma-ester d'alkyle ou d'aryle de l'acide aspartique, delta-ester d'alkyle ou d'aryle de l'acide glutamique, etc...

Dans la formule I, X représente notamment un groupement $X_1 = -NH-R_2$ dérivant d'une amine $R_2-NH_2$ qui peut être par exemple une amine aromatique telle que la p-nitroaniline, l'alpha-naphtylamine, la beta-naphtylamine, la 4-méthoxy-beta-naphtylamine, l'amino-7-méthyl-4-coumarine, l'amino-7-trifluorométhyl-4-coumarine,

l'amino-7-nitro-4-oxa-2-benzodiazole-1,3, l'amino-3-éthyl-9-carbazole, ou X représente un groupement $X_2$ = $-NH-R_3$ dérivant d'une drogue aminée $R_3-NH_2$ qui peut être par exemple la daunorubicine, la puromycine, l'adriamycine, la primaquine, la chloroquine etc... ou bien X représente un autre groupement actif $X_3$, capable de conférer par exemple une activité pharmacologique au peptide de formule I, $X_3$ étant en particulier un groupement conservant ou améliorant l'activité inhibitrice d'enzyme, comme un groupement $-H$, $-OH$, $-CH_2Cl$, -O-alkyle, -O-Ar (Ar étant un groupement aryle, tel que phényle, éventuellement substitué, par exemple un groupement nitrophényle),

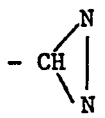 , 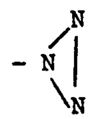 , -O-Alk-Hg-Cl,

ou - NH - Alk - Hg - Cl (Alk étant un groupement alkylène).

Parmi les dérivés de formule I, et sans que cette liste soit limitative, ceux qui comportent les séquences énumérées ci-dessous sont utilisables comme substrats, y compris comme substrats-suicides, pour les enzymes suivantes (étant entendu que les séquences indiquées sont les séquences minimales et qu'il est possible d'insérer 1 à 3 autres acides aminés entre le groupement PHA et le peptide indiqué) :

La chymotrypsine :

PHA-L-Phé-X
PHA-L-Tyr-X
PHA-L-Ala-L-Ala-L-Phé-X
PHA-Gly-Gly-L-Pro-L-Phé-X

La trypsine :

PHA-L-Arg-X
PHA-L-Lys-X
PHA-L-Phé-L-Val-L-Arg-X
PHA-L-Pro-L-Phé-L-Arg-X
PHA-L-Phé-L-Ser-L-Arg-X
PHA-L-Val-L-Leu-Gly-L-Arg-X

La thrombine ou la plasmine :

PHA-L-Phé-L-Val-L-Arg-X
PHA-Gly-L-Pro-L-Arg-X

La plasmine :

PHA-L-Ala-L-Ala-L-Lys-X
PHA-L-Val-L-Leu-L-Lys-X
PHA-L-Ile-L-Phé-L-Lys-X
PHA-L-Phé-L-Ala-L-Lys-X
PHA-L-Phé-L-Val-L-Arg-X

L'élastase :

PHA-L-Ala--Ala-L-Ala-X

PHA-L-Ala-L-Ala-L-Pro-L-Val-X

PHA-L-Ala-L-Ala-L-Pro-L-Ala-X

PHA-L-Ala-L-Ala-L-Pro-L-Phé-X

PHA-L-Ala-L-Ala-L-Pro-L-Mét-X

La cathepsine B :

PHA-L-Phé-L-Val-L-Arg-X

La cathepsine D :

PHA-L-Arg-Gly-L-Phé-L-Pro-X

PHA-L-Arg-L-Phé-L-Pro-X

PHA-L-Ala-L-Arg-L-Arg-X

La cathepsine G :

PHA-L-Ala-L-Ala-L-Pro-L-Mét-X

PHA-L-Phé-L-Leu-L-Phé-X

La papaïne:

PHA-L-Arg-X

La ficine ou les peptidases neutres:

PHA-Gly-Gly-L-Leu-X

La collagénase:

PHA-Gly-L-Pro-L-Ala-Gly-L-Pro-X

PHA-L-Pro-L-Ala-Gly-L-Pro-X

L'urokinase ou l'activateur du plasminogène:

PHA-Gly-Gly-L-Arg-X

PHA-L-Pro-L-Phé-L-Arg-X

PHA-L-Phé-Gly-Gly-L-Arg-X

La kallikréine ou l'activateur du plasminogène:

PHA-L-Pro-L-Phé-L-Arg-X

Le facteur $X_a$:

PHA-L-Ile-Gly-Gly-L-Arg-X

La protéase de Limulus polyphemus activée par un lipopolysaccharide:

PHA-L-Val-L-Leu-Gly-L-Arg-X

Dans les listes de dérivés peptidiques données ci-dessus, chaque acide aminé est représenté par son symbole usuel. D'une façon générale, dans les formules de la présente demande, on a supposé par convention que chaque acide aminé a son groupement N-terminal à gauche et son groupement C-terminal à droite.

Dans les listes de substrats données ci-dessus, X peut prendre notamment toutes les valeurs désignées plus haut par $X_2$ ou $X_3$ (les produits sont alors utilisables comme médicaments) ou par $X_1$ (les produits sont

alors utilisables notamment pour effectuer des dosages).

Parmi les dérivés de formule I et sans que cette liste soit limitative, ceux qui sont énumérés ci-dessous sont utilisables comme prodrogues anti-tumorales. Ils présentent l'avantage de ne libérer la drogue qu'au voisinage des cellules tumorales, sous l'action des protéases sécrétées par celles-ci.

PHA-L-Ala-L-Ala-L-Pro-L-Ala-Daunorubicine
PHA-L-Ala-L-Ala-L-Pro-L-Ala-L-Leu-Daunorubicine
PHA-L-Ala-L-Ala-L-Pro-L-Ala-Adriamycine
PHA-L-Ala-L-Ala-L-Pro-L-Ala-L-Leu-Adriamycine
PHA-L-Ala-L-Ala-L-Pro-L-Val-Daunorubicine
PHA-L-Ala-L-Ala-L-Pro-L-Val-L-Leu-Daunorubicine
PHA-L-Ala-L-Ala-L-Pro-L-Val-Adriamycine
PHA-L-Ala-L-Ala-L-Pro-L-Val-L-Leu-Adriamycine
PHA-L-Phé-L-Val-L-Arg-Daunorubicine
PHA-L-Phé-L-Val-L-Arg-L-Leu-Daunorubicine
PHA-L-Phé-L-Val-L-Arg-Adriamycine
PHA-L-Phé-L-Val-L-Arg-L-Leu-Adriamycine
PHA-L-Phé-L-Ala-L-Lys-Daunorubicine
PHA-L-Phé-L-Ala-L-Lys-L-Leu-Daunorubicine
PHA-L-Phé-L-Ala-L-Lys-Adriamycine
PHA-L-Phé-L-Ala-L-Lys-L-Leu-Adriamycine
PHA-Gly-Gly-L-Arg-Daunorubicine
PHA-Gly-Gly-L-Arg-L-Leu-Daunorubicine
PHA-Gly-Gly-L-Arg-Adriamycine
PHA-Gly-Gly-L-Arg-L-Leu-Adriamycine
PHA-L-Pro-L-Phé-L-Arg-Daunorubicine
PHA-L-Pro-L-Phé-L-Arg-L-Leu-Daunorubicine
PHA-L-Pro-L-Phé-L-Arg-Adriamycine
PHA-L-Pro-L-Phé-L-Arg-L-Leu-Adriamycine

Parmi les dérivés de formule I utilisables comme prodrogues antiparasitaires, on citera à titre d'exemples non limitatifs les composés suivants :

PHA-L-Val-L-Leu-Gly-L-Arg-Primaquine
PHA-L-Val-L-Leu-Gly-L-Arg-Chloroquine

Il a été trouvé, conformément à la présente invention, que les nouveaux dérivés de formule I portant un groupement polyhydroxyalcanoyle en position N-terminale sont solubles à très haute concentration dans l'eau ou dans un tampon. Il n'est donc plus nécessaire de dissoudre préalablement ces dérivés (substrats des protéases ou peptidases) dans un

solvant organique. Grâce à ces dérivés, la sensibilité des méthodes de détection des protéases et peptidases est considérablement augmentée et il est en outre possible de déterminer facilement le meilleur substrat peptidique pour une protéase donnée, comme cela est illustré par l'exemple d'application B ci-après. En effet, pour que la vitesse d'hydrolyse soit élevée, il faut que la concentration de substrat libre soit la plus élevée possible, par exemple nettement supérieure à la constante de Michaelis. Avec les protéases et peptidases, cette constante est de l'ordre de grandeur de 1mM. Lorsque le substrat est peu soluble, il se trouve sous forme d'une dispersion d'agrégats dans le tampon et la concentration efficace (substrat non agrégé) est très inférieure à la valeur de la constante de Michaelis. En outre, les dérivés selon la présente invention, qui sont très hydrosolubles, ne portent pas de charges dues au substituant solubilisant, contrairement aux dérivés dont le groupement N-terminal est substitué par des restes succinyle ou glutaryle. Enfin la préparation des dérivés de l'invention est particulièrement simple: le peptide ou l'amino-acide est substitué par réaction avec la lactone ou un ester actif du PHA sur son groupement alpha-aminé, puis le groupement alpha-carboxylique du peptide ou de l'amino-acide est substitué par un groupement actif convenable.

L'invention a également pour objet un procédé de préparation des nouveaux dérivés acylés hydrosolubles décrits ci-dessus. Ce procédé est principalement caractérisé par le fait que l'on soumet un acide aminé ou un peptide, ou un de leurs dérivés à groupement C-terminal modifié, à une réaction d'acylation du groupement N-terminal par un agent d'acylation dérivé d'un acide polyhydroxyalcanoïque.

L'invention a en particulier pour objet un procédé de préparation des nouveaux dérivés de formule I. Ce procédé est caractérisé par le fait que l'on utilise comme produit de départ un dérivé d'amino-acide ou un peptide de formule:

$$H-(NH-\underset{\displaystyle R}{\underset{|}{C}H}-CO)_n-X \qquad II$$

dans laquelle:

n, R et X sont définis comme précédemment, que l'on fait réagir le composé de formule II avec un dérivé actif d'un acide polyhydroxyalcanoïque de formule :

$$R_1-(CHOH)_m-COOH \qquad III$$

dans laquelle:

$R_1$ et m sont définis comme précédemment,

pour obtenir un composé de formule:

$$R_1 - (CHOH)_m-CO-(NH-\underset{R}{\underset{|}{CH}}-CO)_n-X \qquad IV$$

que l'on transforme, si désiré, en tout autre dérivé de formule I selon les méthodes connues.

D'une façon générale, les diverses transformations d'un composé de formule I en un autre composé de formule I (avec un substituant X différent) sont effectuées selon les méthodes classiques décrites dans les ouvrages spécialisés tels que par exemple "Methods in Enzymology" - Proteolytic Enzymes, en particulier Volumes 19, 45 et 80.

Les peptides de départ peuvent être soit achetés dans le commerce, soit synthétisés selon les méthodes classiques de la synthèse peptidique ; (voir par exemple Cross, E. and Meienhoffer J. (1979) The peptides : Analysis, synthesis, biology. Academic Press, New York, Volumes I, II, III et suivants).

Dans des modes d'exécution particuliers, le procédé de l'invention peut encore présenter les caractéristiques suivantes prises isolément ou en combinaison:

- le dérivé actif d'acide polyhydroxyalcanoïque est un dérivé capable d'acyler le groupement N-terminal du composé II. Ce dérivé actif est par exemple la lactone correspondant au dit acide ou un ester actif dudit acide tel que par exemple l'ester de N-hydroxysuccinimide.

- pour transformer le composé de formule IV (avec X = OH) en dérivé de formule I dans laquelle X représente -NH- $R_2$ ou -NH- $R_3$, on fait réagir l'amine $R_2$-$NH_2$ ou $R_3$-$NH_2$ en présence d'un agent de couplage tel que par exemple le benzotriazol-1-yl-oxy-tris (diméthylamino) phosphonium hexafluorophosphate (ou BOP).

L'invention a également pour objet l'utilisation comme dérivés hydrosolubles, de composés choisis parmi les acides aminés, les peptides, les acides aminés à groupement C-terminal modifié et les peptides à groupement C-terminal modifié, de dérivés (N-terminal)-acylés de ces composés, cette utilisation étant caractérisée par le fait que le groupement N-terminal desdits composés est acylé par un groupement polyhydroxyalcanoïque.

L'invention a notamment pour objet l'utilisation comme médicaments hydrosolubles des dérivés acylés tels que définis ci-dessus possédant une activité pharmacologique, en particulier des dérivés de formule I pour lesquels X = NH-$R_3$, $R_3$ représentant le reste d'une drogue aminée ou bien X représente un groupement $X_3$ choisi parmi -H, -OH, -$CH_2$Cl, -O- alkyle, -O-Ar (Ar étant un groupement aryle, tel que phényle, éventuellement substitué, par exemple un groupement nitrophényle),

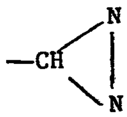 , 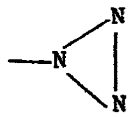 , -O-Alk-Hg-Cl,

ou -NH-Alk-Hg-Cl (Alk étant un groupement alkylène).

Bien entendu, l'invention s'étend aux compositions pharmaceutiques contenant au moins un composé de formule I comme ingrédient actif, éventuellement en association avec un excipient approprié.

Lorsque les dérivés de formule I sont des prodrogues ils sont utilisés à des doses correspondant aux doses usuelles de la drogue active avec les mêmes indications thérapeutiques que ladite drogue active.

Généralement, la toxicité des prodrogues est inférieure à celle des drogues correspondantes.

Lorsque les médicaments de l'invention sont des inhibiteurs d'enzyme ils sont utilisés, notamment comme agents de traitement préventif de la formation de métastases et de la prolifération des tumeurs. Les doses efficaces varient généralement de 0,1 à 10 mg/Kg.

Les médicaments de l'invention sont administrés par toute voie convenable. Il faut noter toutefois que la voie orale ne convient généralement pas.

Ils peuvent être présentés notamment sous forme de solutions injectables, de poudres lyophilisées à diluer dans le sérum physiologique, de solutions ou d'émulsions à appliquer sur la peau ou les muqueuses, de solutions en conditionnement pressurisé pour aérosols, etc...

Les exemples suivants illustrent l'invention sans la limiter.

EXEMPLE 1 : Substrat de l'élastase

Le peptide L-Ala-L-Ala-L-Pro-L-Ala (1 mmole) synthétisé par les méthodes classiques de la synthèse peptidique est dissous dans le diméthylsulfoxyde (5ml) en présence de N-triméthylbenzylamine (sous forme de méthoxyde de N-triméthylbenzylammonium) (2mmoles). A cette solution, on ajoute la glucono -delta-lactone (2mmoles). La solution est agitée à 50°C pendant 15 heures.

Le PHA-peptide est purifié par chromatographie sur une colonne de silice (3x30cm) dans le système-solvant ($CHCl_3/CH_3OH/H_2O/CH_3CO_2H$, 8:6:1:1 en volume). La solution contenant le PHA-peptide est évaporée à siccité. Le résidu (huile) est repris dans le N-diméthylformamide (3ml) à 4°C.

A une solution du PHA-peptide (1mmole) dans le N-diméthylformamide sec et dépourvu d'amine (2ml), on ajoute l'hydroxy-1-benzotriazole (1mmole), le dicyclohexylcarbodiimide (1mmole) et l'A.E.C (amino-3-éthyl-9-carbazole) (1mmole). La solution est agitée à 4°C pendant 2h

puis à 25°C pendant 18h. La solution est ensuite refroidie à 4°C pendant 1h pour favoriser la précipitation de la dicyclohexylurée qui est éliminée ensuite par filtration. Le N-diméthylformamide est évaporé sous pression réduite à 50°C. Le PHA-L-Ala-L-Ala-L-Pro-L-Ala-AEC est purifié par chromatographie sur une colonne de gel de silice (3x30cm) équilibré dans le système solvant $CHCl_3/CH_3OH/CH_3CO_2H$, 80:30:5 par volume. Le PHA-L-Ala-L-Ala-L-Pro-L-Ala-AEC est détecté par son absorbance à 340nm. Sa pureté est vérifiée par chromatographie en couche mince. Les fractions contenant le composé sont concentrées à siccité. Le résidu est repris dans 2ml d'eau et la solution est lyophilisée (Rendement: 80 p 100).

Le PHA-L-Ala-L-Ala-L-Pro-L-Ala-AEC est caractérisé en outre par spectrométrie infrarouge ; bandes caractéristiques en $cm^{-1}$ : hydroxyle=3320; amide I:1640; amide II:1535; groupements aromatiques:1490; par spectrophotométrie ultraviolette: maximum d'absorption à 340nm, minimum à 315nm; par spectrofluorimétrie: longueur d'onde maximale d'excitation:342nm, longueur d'onde maximale d'émission : 390nm.

La solubilité du PHA-L-Ala-L-Ala-L-Pro-L-Ala-AEC est supérieure à 80mg/ml dans l'eau distillée ou un tampon de pH compris entre 3 et 9.

EXEMPLE 2: Substrat de l'élastase

Le peptide L-Ala-L-Ala-L-Pro-L-Val-OBzl est synthétisé par les méthodes classiques de la synthèse peptidique. Le symbole Bzl est le symbole classique désignant le dérivé ester benzylique. Le groupement ester benzylique a été choisi pour protéger l'extrémité C-terminale du peptide pendant la synthèse peptidique, et pour permettre une détection simple des peptides et de leurs dérivés au cours des étapes de purification par chromatographie.

Le peptide L-Ala-L-Ala-L-Pro-L-Val-OBzl, HCl (2mmoles) est dissous dans le diméthylsulfoxyde (3ml). A cette solution, on ajoute la triéthylamine (6mmoles) et la glucono-delta-lactone (6mmoles). La solution est agitée à 50°C pendant 15h.

Le PHA-peptide-OBzl est purifié sur une colonne (3x30cm) de silice équilibrée avec le système solvant $CHCl_3/CH_3OH/CH_3CO_2H$: 50:20:1 en volume. Les fractions contenant le PHA-peptide sont rassemblées, le solvant est évaporé sous pression réduite.

Le résidu (huile) est repris dans le méthanol (6ml) contenant 10% d'eau et hydrogénolysé pendant 4h en présence de 200mg de charbon palladié (10% de Pd). Le mélange réactionnel est filtré et évaporé à siccité.

Le PHA-peptide-OH (1mmole) est dissous dans le N-diméthylformamide (2ml). A cette solution, on ajoute l'hydroxy-1-benzotriazole (1mmole), le dicyclohexylcarbodiimide (1mmole) et l'amino-3-éthyl-9-carbazole (1mmole). La

solution est agitée pendant 2h à 4°C puis 18h à 25°C sous atmosphère inerte. Ensuite, la solution est refroidie pour favoriser la précipitation complète de la dicyclohexylurée qui est éliminée par filtration. Le N-diméthylformamide est évaporé sous vide et le PHA-peptide-AEC est purifié par chromatographie sur une colonne (3x30cm) de gel de silice équilibré avec le système-solvant $CHCl_3/CH_3OH/CH_3CO_2H$, 80:30:5 par volume (Rendement : 80 p 100).

Le PHA-L-Ala-L-Ala-L-Pro-L-Val-AEC présente les caractéristiques correspondant au groupement PHA, aux liaisons peptidiques et au groupement AEC, comme indiqué à l'exemple 1.

EXEMPLE 3 : Substrat de la chymotrypsine.

Le chlorhydrate de l'ester méthylique de la L-phénylalanine, L-Phé-$OCH_3$,HCl (2mmoles) est dissous dans le diméthylsulfoxyde (3ml). A cette solution, on ajoute la glucono-delta-lactone (6mmoles) et la N-triéthylamine (6mmoles). La solution est agitée à 50°C pendant 15h. Le PHA-L-Phé-$OCH_3$ est purifié par chromatographie sur une colonne (3x30cm) de gel de silice équilibré dans le système-solvant ($CHCl_3/CH_3OH/H_2O/CH_3CO_2H$, 8:6:1:1 par volume. Le PHA-L-Phé-$OCH_3$ est ensuite saponifié dans une solution de méthanol/eau (1:1, v/v) amenée à pH 10.5 par addition de soude 2N pendant 12h. Le PHA-L-Phé-OH est purifié sur une colonne (3x30cm) de gel de silice équilibré dans le système-solvant $CHCl_3/CH_3OH/CH_3CO_2H/H_2O$, 6:6:1:1. Le PHA-L-Phé-OH (1mmole) est substitué par l'AEC dans les conditions décrites à l'exemple 1.

Le PHA-L-Phé-AEC présente les caractéristiques correspondant au groupement PHA, aux noyaux aromatiques de l'AEC et de Phé, ainsi qu'aux liaisons amides, comme indiqué à l'exemple 1.

EXEMPLE 4 : Substrat de la collagénase

De manière analogue à celle décrite à l'exemple 1, le peptide L-Pro-L-Ala-Gly-L-Pro-OBzL,HCl (synthétisé par les méthodes classiques de la synthèse peptidique) est converti en dérivé PHA-L-Pro-L-Ala-Gly-L-Pro-AEC. Rendement : 60 p 100.

Le PHA-L-Pro-L-Ala-Gly-L-Pro-AEC présente les caractéristiques correspondant au groupe PHA, aux liaisons peptidiques et au groupe AEC, comme indiqué à l'exemple 1.

EXEMPLE 5: Autre substrat de la collagénase

De manière analogue, le dérivé Gly-L-Pro-L-Ala-Gly-L-Pro-OBzl,HCl (synthétisé par les méthodes classiques de la synthèse peptidique) est converti en dérivé PHA-Gly-L-Pro-L-Ala-Gly-L-Pro-AEC. Rendement 60 %.

Le PHA-Gly-L-Pro-L-Ala-Gly-L-Pro-AEC présente les caractéristiques correspondant au groupe PHA, aux liaisons peptidiques et au groupe AEC, comme

indiqué à l'exemple 1.

EXEMPLE 6: Prodrogue

Le PHA-L-Ala-L-Ala-L-Pro-L-Val préparé comme décrit dans l'exemple 2 est couplé à la daunorubicine dans les mêmes conditions que pour l'AEC.

Le dérivé PHA-L-Ala-L-Ala-L-Pro-L-Val-daunorubicine présente les caractéristiques correspondant au groupement PHA, aux liaisons peptidiques et à l'aglycone de la daunorubicine déterminées par spectroscopie infrarouge, absorptiométrie et fluorimétrie.

De manière analogue, on prépare le dérivé PHA-L-Ala-L-Ala-L-Pro-L-Val-L-Leu-Daunorubicine, utilisable comme prodrogue.

EXEMPLE 7 : Prodrogues

De façon analogue, les dérivés PHA-L-Ala-L-Ala-L-Pro-L-Val-adriamycine et PHA-L-Ala-L-Ala-L-Pro-L-Val-L-Leu-adriamycine sont préparés comme le dérivé de la daunorubicine décrit dans l'exemple 6.

EXEMPLE 8 :

De façon analogue, on prépare les composés :

PHA-L-Phé-L-Pro-L-Arg-Adriamycine

PHA-L-Phé-L-Pro-L-Arg-Daunorubicine

PHA-L-Phé-L-Pro-L-Arg-L-Leu-Adriamycine

PHA-L-Phé-L-Pro-L-Arg-L-Leu-Daunorubicine, qui sont utilisables comme prodrogues.

EXEMPLE 9 :

De façon analogue, on prépare le composé :

PHA-L-Val-L-Leu-Gly-L-Arg-Primaquine qui est utilisable comme prodrogue.

EXEMPLES D'APPLICATION

A. Dosage de l'élastase

a) Dosage cinétique

Le peptide PHA-L-Ala-LAla-L-Pro-L-Ala-AEC est dissous dans un tampon Tris-HCl 0.05M, pH 8.8, afin d'obtenir une densité optique à 340nm de 0.1 pour 1cm de trajet optique ($4.3x10^{-5}$M). La solution ainsi obtenue est portée à 37°C et l'on ajoute 10 microlitres d'une solution d'élastase (1,0 à 100 microgrammes/ml) à 1ml de la solution de peptide dans une cuvette de fluorimétrie. La cuvette est introduite immédiatement dans le spectrofluorimètre dont le compartiment échantillon est thermostaté à 37°C. La longueur d'onde d'excitation est 352nm, la longueur d'onde d'émission est 460nm. On enregistre le signal "émission" pendant 3min. L'activité de l'enzyme est déterminée par la pente de la courbe obtenue. Les valeurs de la pente

correspondant à 10ng, 50ng, 100ng, 200ng, 500ng et 1 microgramme d'élastase sont reportées sur un graphique. Dans ces conditions, l'intensité de fluorescence mesurée est proportionnelle à la quantité d'enzyme ajoutée au milieu réactionnel.

b) Dosage classique

La peptide PHA-L-Ala-L-Ala-L-Pro-L-Ala-AEC est dissous dans un tampon Tris-HCl 0.05, pH 8.8 afin d'obtenir une densité optique à 340nm de 0,1 pour un trajet optique de 1cm. Cette solution est répartie dans des tubes à hémolyse siliconés (1ml par tube). Dans chaque tube, on ajoute 10 microlitres d'une solution d'élastase dans un tampon Tris-HCl, pH 8.8 contenant 0/0,5/1/2/5/10ng d'élastase. Les tubes sont maintenus à 37°C pendant 30min. La réaction est arrêtée par addition de 0,3ml de N-diméthylformamide contenant du chlorure de benzamidinium (40mM). L'intensité de fluorescence est mesurée à 460nm; la longueur d'onde d'excitation est 352nm. Les intensités obtenues corrigées de l'intensité du blanc, sont portées sur un graphique en fonction de la concentration d'élastase. Dans ces conditions, l'intensité de fluorescence corrigée est proportionnelle à la concentration en élastase. Ainsi, il est possible de mettre en évidence des concentrations aussi faibles qu'un nanogramme d'élastase par ml, soit une concentration 40 picomolaire ou encore $1,2x10^{-7}$ U/ml.

c) Constante de Michaelis

La constante de Michaelis correspondant au peptide PHA-L-Ala-L-Ala-L-Pro-L-Ala-AEC est déterminée par la mesure de l'activité de l'élastase (10ng/ml) mise en présence du peptide à des concentrations comprises entre $4,2x10^{-4}$ M et $4,3x10^{-5}$ M. Les intensités de fluorescence obtenues après 30min à 37°C sont utilisées pour tracer un diagramme de Lineweaver-Burck. La droite obtenue permet de déterminer la constante de Michaelis qui est égale à 0,3mM.

B. Dosage d'une protéase du Plasmodium falciparum

Le principe de ce dosage est donné ci-après :

Des érythrocytes infestés par le parasite sont lysés par un agent tensio-actif. A ce lysat, on ajoute le dérivé PHA-peptidyl-AEC et la solution est incubée à 37°C pendant 45 minutes. Ensuite, l'AEC libéré par une protéase du parasite est extrait par l'acétate d'éthyle, et le dosage de l'AEC libéré est effectué par spectrofluorimétrie (M. Monsigny et al. The EMBO J., 1, 303-306 (1982)).

En utilisant cette méthode avec divers peptides, il a été trouvé que le peptide présent dans le dérivé PHA-L-Val-L-Leu-Gly-L-Arg-AEC

était le substrat peptidique le plus facilement hydrolysé par les protéases du parasite.

Dans ces expériences, l'agent tensio-actif était le gluconoylaminododécane.

C. Propriétés des prodrogues

A titre d'exemple, le peptide PHA-L-Ala-L-Ala-L-Pro-L-Val-L-Leu-Adriamycine est soluble dans l'eau. Il est hydrolysé par l'élastase leucocytaire ou les protéases d'origine tumorale en libérant la drogue active : L-Leu-Adriamycine. La prodrogue ne pénètre pas dans les cellules normales (lymphocyte) ou tumorales (cellules leucémiques L1210, ou cellules du carcinome pulmonaire de Lewis "LLC"). Au contraire, la drogue active L-Leu-Adriamycine libérée par l'action des protéases, pénètre dans les cellules tumorales et provoque la mort de ces cellules.

Le peptide PHA-L-Phé-L-Pro-L-Arg-L-Leu-Adriamycine est soluble dans l'eau. Il est hydrolysé par la trypsine et par la plasmine en libérant la drogue active L-Leu-Adriamycine. Cette prodrogue se comporte vis à vis des cellules tumorales comme la prodrogue décrite ci-dessus.

REVENDICATIONS

1. Dérivés acylés hydrosolubles de peptides, d'amino-acides, ou de dérivés de peptides ou d'amino-acides à groupement C-terminal modifié, caractérisés par le fait que leur groupement N-terminal est acylé par un groupement polyhydroxyalcanoyle.

2. Dérivés selon la revendication 1, caractérisés par le fait que ledit groupement polyhydroxyalcanoyle possède 3 à 10 atomes de carbone et au moins deux groupements hydroxyles.

3. Dérivés selon l'une quelconque des revendications précédentes caractérisés par le fait que leur groupement C-terminal porte un substituant -X capable de conférer auxdits peptides ou amino-acides une coloration, une fluorescence ou une activité pharmacologique.

4. Dérivés acylés selon l'une quelconque des revendications précédentes, caractérisés par le fait que le groupement polyhydroxyalcanoyle est choisi dans le groupe constitué par les groupements dihydroxypropionyle, érythronoyle, thréonoyle, ribonoyle, arabinoyle, xylonoyle, lyxonoyle, gluconoyle, galactonoyle, mannonoyle, glycoheptonoyle et glycooctonoyle.

5. Dérivés acylés selon l'une quelconque des revendications précédentes caractérisés par le fait qu'ils répondent à la formule générale I

$$R_1 - (CHOH)_m\text{-CO-}(\text{NH-}\underset{\substack{| \\ R}}{\text{CH}}\text{-CO})_n\text{-X} \qquad (I)$$

dans laquelle $R_1$ représente -H ou $-CH_3$, et m est un nombre entier pouvant varier de 2 à 7, R est une chaîne latérale d'amino-acides, n est un nombre entier pouvant varier de 1 à 10 et X est un substituant capable de conférer aux dérivés de formule I une coloration, une fluorescence ou une activité pharmacologique, étant entendu que lorsque n est supérieur à 1, les substituants R peuvent être différents dans un même composé de formule I.

6. Dérivés acylés selon la revendication 5, caractérisés par le fait que R représente la chaîne latérale d'amino-acides choisis parmi les suivants sérine, thréonine, asparagine, proline, glutamine, glycine, alanine, valine, cystéine ou un de ses dérivés S-substitués, méthionine, leucine, isoleucine, phénylalanine, p-nitrophénylalanine, tyrosine ou un de ses dérivés O-substitués, lysine, ornithine, arginine, tryptophane, acide aspartique, acide glutamique, gamma-ester d'alkyle ou d'aryle de l'acide aspartique, et delta-ester d'alkyle ou d'aryle de l'acide glutamique.

7. Dérivés acylés selon l'une quelconque des revendications 3 à 6, caractérisés par le fait que X représente un groupement $-NH-R_2$ dérivant d'une amine aromatique $R_2-NH_2$, un groupement $-NH-R_3$ dérivant d'une drogue aminée $R_3-NH_2$, ou bien X représente un groupement actif $X_3$ capable de conférer une activité pharmacologique audit peptide ou amino-acide.

8. Dérivés acylés selon la revendication 7, caractérisés par le fait que ladite amine aromatique est choisie dans le groupe constitué par la p-nitroaniline, l'alpha-naphtylamine, la beta-naphtylamine, la 4-méthoxy-beta-naphtylamine, l'amino-7-méthyl-4-coumarine, l'amino-7-trifluorométhyl-4-coumarine, l'amino-7-nitro-4-oxa-2-benzodiazole-1,3 et l'amino-3-éthyl-9-carbazole.

9. Dérivés acylés selon la revendication 7, caractérisés par le fait que ladite drogue aminée $R_3-NH_2$ est choisie dans le groupe constitué par la daunorubicine, la puromycine, l'adriamycine, la primaquine et la chloroquine.

10. Dérivés acylés selon la revendication 7, caractérisés par le fait que ledit groupement $X_3$ est un groupement -H, -OH, $-CH_2Cl$, -O-alkyle, -O-Ar (Ar étant un groupement aryle éventuellement substitué),

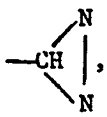, 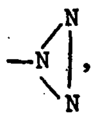, -O-Alk-Hg-Cl,

ou -NH-Alk-Hg-Cl, Alk étant un groupement alkylène.

11. Dérivés selon l'une quelconque des revendications 7 à 10, caractérisés par le fait qu'ils sont choisis parmi les composés suivants:

- PHA-L-Phé-X
- PHA-L-Tyr-X
- PHA-L-Ala-L-Ala-L-Phé-X
- PHA-Gly-Gly-L-Pro-L-Phé-X
- PHA-L-Arg-X
- PHA-L-Lys-X
- PHA-L-Phé-L-Val-L-Arg-X
- PHA-L-Pro-L-Phé-L-Arg-X
- PHA-L-Phé-L-Ser-L-Arg-X
- PHA-L-Val-L-Leu-Gly-L-Arg-X
- PHA-L-Phé-L-Val-L-Arg-X
- PHA-Gly-L-Pro-L-Arg-X
- PHA-L-Ala-L-Ala-L-Lys-X
- PHA-L-Val-L-Leu-L-Lys-X
- PHA-L-Ile-L-Phé-L-Lys-X
- PHA-L-Phé-L-Ala-L-Lys-X
- PHA-L-Phé-L-Val-L-Arg-X
- PHA-L-Ala-L-Ala-L-Ala-X
- PHA-L-Ala-L-Ala-L-Pro-L-Val-X
- PHA-L-Ala-L-Ala-L-Pro-L-Ala-X
- PHA-L-Ala-L-Ala-L-Pro-L-Phé-X
- PHA-L-Ala-L-Ala-L-Pro-L-Mét-X

- PHA-L-Phé-L-Val-L-Arg-X
- PHA-L-Arg-Gly-L-Phé-L-Pro-X
- PHA-L-Arg-L-Phé-L-Pro-X
- PHA-L-Ala-L-Arg-L-Arg-X
- PHA-L-Ala-L-Ala-L-Pro-L-Mét-X
- PHA-L-Phé-L-Leu-L-Phé-X
- PHA-L-Arg-X
- PHA-Gly-Gly-L-Leu-X
- PHA-Gly-L-Pro-L-Ala-Gly-L-Pro-X
- PHA-L-Pro-L-Ala-Gly-L-Pro-X
- PHA-Gly-Gly-L-Arg-X
- PHA-L-Pro-L-Phé-L-Arg-X
- PHA-L-Phé-Gly-Gly-L-Arg-X
- PHA-L-Pro-L-Phé-L-Arg-X
- PHA-L-Ile-Gly-Gly-L-Arg-X
- PHA-L-Val-L-Leu-Gly-L-Arg-X,

dans lesquelles PHA symbolise ledit groupement polyhydroxyalcanoyle et X est défini comme dans l'une quelconque des revendications 3, 5 et 7 à 10.

12. Dérivés acylés selon la revendication 9, caractérisés par le fait qu'ils sont choisis parmi les dérivés suivants :

- PHA-L-Ala-L-Ala-L-Pro-L-Ala-Daunorubicine
- PHA-L-Ala-L-Ala-L-Pro-L-Ala-L-Leu-Daunorubicine
- PHA-L-Ala-L-Ala-L-Pro-L-Ala-Adriamycine
- PHA-L-Ala-L-Ala-L-Pro-L-Ala-L-Leu-Adriamycine
- PHA-L-Ala-L-Ala-L-Pro-L-Val-Daunorubicine
- PHA-L-Ala-L-Ala-L-Pro-L-Val-L-Leu-Daunorubicine
- PHA-L-Ala-L-Ala-L-Pro-L-Val-Adriamycine
- PHA-L-Ala-L-Ala-L-Pro-L-Val-L-Leu-Adriamycine
- PHA-L-Phé-L-Val-L-Arg-Daunorubicine
- PHA-L-Phé-L-Val-L-Arg-L-Leu-Daunorubicine
- PHA-L-Phé-L-Val-L-Arg-Adriamycine
- PHA-L-Phé-L-Val-L-Arg-L-Leu-Adriamycine
- PHA-L-Phé-L-Ala-L-Lys-Daunorubicine
- PHA-L-Phé-L-Ala-L-Lys-L-Leu-Daunorubicine
- PHA-L-Phé-L-Ala-L-Lys-Adriamycine
- PHA-L-Phé-L-Ala-L-Lys-L-Leu-Adriamycine
- PHA-Gly-Gly-L-Arg-Daunorubicine
- PHA-Gly-Gly-L-Arg-L-Leu-Daunorubicine
- PHA-Gly-Gly-L-Arg-Adriamycine
- PHA-Gly-Gly-L-Arg-L-Leu-Adriamycine

- PHA-L-Pro-L-Phé-L-Arg-Daunorubicine
- PHA-L-Pro-L-Phé-L-Arg-L-Leu-Daunorubicine
- PHA-L-Pro-L-Phé-L-Arg-Adriamycine
- PHA-L-Pro-L-Phé-L-Arg-L-Leu-Adriamycine
- PHA-L-Val-L-Leu-Gly-L-Arg-Primaquine
- PHA-L-Val-L-Leu-Gly-L-Arg-Chloroquine

13. Procédé de préparation des dérivés acylés selon l'une quelconque des revendications précédentes caractérisé par le fait que l'on soumet un acide aminé ou un peptide, ou un de leurs dérivés à groupement C-terminal modifié, à une réaction d'acylation du groupement N-terminal par un agent d'acylation dérivé d'un acide polyhydroxyalcanoïque.

14. Procédé selon la revendication 13, caractérisé par le fait que l'on utilise comme produit de départ un dérivé d'amino-acide ou un peptide de formule $H-(NH-\underset{R}{\underset{|}{C}H}-CO)_n-X$ II

dans laquelle n, R et X sont définis comme dans l'une quelconque des revendications 3, 5 et 7 à 10, que l'on fait réagir le composé de formule II avec un dérivé actif d'un acide polyhydroxyalcanoïque de formule :

$$R_1-(CHOH)_m-COOH \qquad III$$

dans laquelle :

$R_1$ et m sont définis comme précédemment,
pour obtenir un composé de formule :

$$R_1-(CHOH)_m-CO-(NH-\underset{R}{\underset{|}{C}H}-CO)_n-X \qquad IV$$

que l'on transforme, si désiré, en tout autre dérivé de formule I selon les méthodes connues.

15. Procédé selon la revendication 14, caractérisé par le fait que ledit dérivé actif est la lactone ou un ester de N-hydroxysuccinimide du dit acide polyhydroxyalcanoïque.

16. Utilisation, comme dérivés hydrosolubles de composés choisis parmi les peptides, les acides-aminés à groupement C-terminal modifié et les peptides à groupement C-terminal modifié, de dérivés (N-terminal)-acylés de ces composés, le groupement N-terminal desdits composés étant acylé par un groupement polyhydroxyalcanoïque.

17. Utilisation selon la revendication 16, caractérisée par le fait que ledit dérivé hydrosoluble est tel que défini dans l'une quelconque des revendications 2 à 12.

18. Utilisation selon la revendication 16 des composés de formule I pour lesquels X = $NH-R_3$, $R_3$ représentant le reste d'une drogue aminée ou bien X représente un groupement $X_3$ choisi parmi -H, -OH, $-CH_2Cl$, -O- alkyle, -O-Ar

(Ar étant un groupement aryle éventuellement substitué),

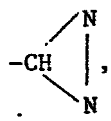, 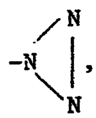, -O-Alk-Hg-Cl,

ou -NH-Alk-Hg-Cl (Alk étant un groupement alkylène), comme médicaments hydrosolubles.

19. Composition pharmaceutique, caractérisée par le fait qu'elle renferme comme ingrédient actif au moins un médicament hydrosoluble tel que défini dans la revendication 18.

Office européen des brevets

0126685

Numéro de la demande

# RAPPORT DE RECHERCHE EUROPEENNE

EP 84 40 0984

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | FR-A-2 398 049 (R. GEDEON)<br>* en entier * | 1,3,5,<br>7,19 | C 07 C 103/52<br>A 61 K 37/02<br>C 07 H 15/24 |
| Y | FR-A-2 471 411 (ABBOTT)<br>* en entier * | 1,3,5,<br>7,19 | |
| Y | US-A-4 169 141 (TOPLISS)<br>* page de garde; colonnes 9,10 * | 1,3,5,<br>7,19 | |
| Y | US-A-3 707 559 (MAZUR)<br>* en entier * | 1,3,5,<br>7,19 | |
| Y | BE-A- 885 303 (CONT. PHARMA)<br>* en entier * | 1,3,5,<br>7,19 | DOMAINES TECHNIQUES RECHERCHES (Int. Cl. 3) |
| Y | EP-A-0 007 182 (GOODRICH)<br>* en entier * | 1,3,5,<br>7,19 | C 07 C 103/00<br>A 61 K 37/00<br>C 07 H 15/24 |
| Y | US-A-4 256 632 (LEVIN)<br>* en entier * | 1,3,5,<br>7,19 | |

Le présent rapport de recherche a été établi pour toutes les revendications

| Lieu de la recherche | Date d'achèvement de la recherche | Examinateur |
|---|---|---|
| LA HAYE | 16-08-1984 | RAJIC M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82